# FASCICULE DE BREVET EUROPEEN

(11) **EP 0 649 473 B1**
(45) Date de publication et mention de la délivrance du brevet: **11.06.1997**
(21) Numéro de dépôt: 93916020.6
(22) Date de dépôt: 20.07.1993
(51) Int. Cl.: C12Q 1/04, G01N 33/569

(54) **PROCEDE D'AMPLIFICATION DES AGENTS INFECTIEUX EN DIAGNOSTIC "IN VITRO" SUR CULTURES CELLULAIRES MYCOPLASMISEES**
VERFAHREN ZUR VERMEHRUNG VON INFEKTIONSAGENZIEN IN "IN-VITRO"-DIAGNOSTIK MIT MYKOPLASMA ENTHALTENDEN ZELLENKULTUREN
METHOD FOR AMPLIFYING INFECTIOUS AGENTS FOR (IN VITRO) DIAGNOSIS IN CELL CULTURES CONTAINING MYCOPLASMA

(30) Priorité: 21.07.1992 FR 9209263
(43) Date de publication de la demande: 26.04.1995
(73) Titulaire: INTERNATIONAL MICROBIO, F-83870 Signes (FR)
(72) Inventeur: PAPIEROK, Gérard, F-83400 Hyères (FR); PAUTRAT, Gérard, F-13012 Marseille (FR); ESCARGUEL, Claude, F-83110 Sanary-sur-Mer (FR)
(74) Mandataire: Marek, Pierre, Cabinet Marek
(86) Numéro de dépôt international: FR9300740
(87) Numéro de publication internationale: WO9402630

(56) Documents cités:
- BIOCHEMICAL AND BIOPHYSICAL RESEARCH COMMUNICATIONS vol. 170, no. 3 , 1990 , NEW YORK NY USA pages 1365 - 1370 I.H. CHOWDHURY 'Mycoplasma can enhance HIV replication in vitro; a possible co-factor responsible for the progression of AIDS.'
- DISSERTATION ABSTRACTS INTERNATIONAL B vol. 34, no. 4 , 1973 , PHILADELPHIA PA USA pages 1652 - 1653 W.H. MILLIGAN III 'Effect of mycoplasma pneumoniae on rhinovirus replication in cell culture.'
- ABSTRACTS OF THE GENERAL MEETING OF THE AMERICAN SOCIETY FOR MICROBIOLOGY vol. 93 , 16 Mai 1993 , ATLANTA GA USA page 169 G. PAUTRAT ET AL. 'The MCR mycoplasmal cell reactivity. A new concept in cellular infectivity application as amplification factor for in vitro diagnosis purposes.'
- BIOCHEMICAL & BIOPHYSICAL RESEARCH COMMUNICATIONS, vol. 170, no. 3, 1990, New York, NY (US); I.H. CHOWDHURY, pp. 1365-1370/
- DISSERTATION ABSTRACTS INTERNATIONAL B, vol. 34, no. 4, 1973, Philadelphia, PA (US); W.H. MILLIGAN III, pp. 1652-1653/
- ABSTRACTS OF THE GENERAL MEETING OF THE AMERICAN SOCIETY FOR MICROBIOLOGY, vol. 93, 16 Mai 1993, Atlanta, GA (US); G. PAUTRAT et al., p. 169/

## Description

La présente invention concerne un procédé d'amplification des agents infectieux en diagnostic "in vitro" sur cultures cellulaires mycopLasmisées.

La culture cellulaire est encore la technique de référence pour le diagnostic de nombreuses maladies infectieuses. La spécificité cellulaire souvent associée avec un effet cytopathogène caractéristique, est généralement confirmée par une détection immunologique révélant une structure antigènique spécifique de l'agent infectieux recherché. La culture cellulaire fournit alors une phase d'amplification de cet agent. Par exemple, le HSV (Herpès Simptex Virus) est ainsi recherché sur cellules Véro, le Chlamydiae sur cellules Mac Coy, le RSV (Virus Respiratoire Syncitial) sur les cellules Hep-2, etc.

Certaines infections étant rares et de développement difficile sur cultures cellulaires, d'une part (exemple Chlamydiae), et le germe recherché pouvant être peu abondamment représenté dans le prélèvement, d'autre part, il importe d'optimiser au maximum le système de diagnostic in vitro des agents infectieux correspondants, notamment par un procédé d'amplification préalable du nombre d'agents infectieux. C'est le but de la présente invention, qui a en outre l'intérêt de mettre en évidence le rôle de ce type de cofacteur en pathologie humaine et de compléter un modèle in vitro dans le sens des écosystèmes plus proche de la réalité in vivo.

Longtemps considérés comme opportunistes ou associés à certaines infections, les mycoplasmes ont aussi été suspectés d'être eux-mêmes responsables de pathologies.

Selon le document BIOCHEMICAL AND BIOPHYSICAL RESEARCH COMMUNICATIONS, PAGES 1365-1370, c'est la présence de protéines membranaires des mycoplasmes qui permet d'augmenter la production du virus "HIV" dans les cellules Molt-4- régulièrement infectées par ce dernier.

Ce procédé d'amplification virale nécessite comme agent potentialisateur des fragments membranaires de mycoplasmes. Or, ce matériel inerte est obtenu par intervention humaine selon des techniques biochimiques. Ces fragments et leur utilisation dans le procédé d'amplification ne reflète donc pas la réalité in vivo. Le travail ayant conduit à la présente invention a consisté à étudier in vitro l'impact des mycoplasmes sur d'autres infections, notamment celles où les mycoplasmes étaient considérés comme fréquemment associés (pathologies de la sphère uro-génitale et pulmonaire).

Selon le procédé de l'invention, on infecte volontairement des cellules cultivées "in vitro" avec des mycoplasmes vivants. Cette inoculation permet d'induire une réponse cellulaire potentialisée par la présence du ou des mycoplasmes utilisés.

Selon une application particulièrement intéressante, l'invention concerne la "mycoplasmisation" de cellules, utilisées, entre autre, pour la détection de certains agents infectieux, par des mycoplasmes vivants potentialisant de façon importante l'infectiosité de ces agents, et l'utilisation biotechnologique qui en découle, notamment en terme d'amplification de signal.

Le néologisme "mycoplasmisation" désignera le fait d'infecter volontairement des cellules avec des mycoplasmes vivants, dans un but précis. Ces cellules seront dites "mycoplasmisées", par opposition à l'appellation "cellules mycoplasmées" qui sous-entend que ces dernières ont été contaminées par hasard.

Dans l'application avantageuse susmentionnée, l'invention concerne la mise en évidence et l'utilisation biotechnologique du pouvoir amplificateur du mycoplasme vivant dans la recherche d'agents infectieux inoculés "in vitro" à des cultures de cellules ; cellules et mycoplasmes étant, dans ce cas, choisis en fonction de la sphère où le prélèvement est effectué, et de l'agent recherché.

Plus généralement, le procédé selon l'invention consiste en l'utilisation in vitro d'écosystème amplifiant la technique conventionnelle de culture cellulaire de virus (par exemple: HSV, RSV, etc) ou de bactéries (par exemple : Chlamydiae, Legionella, Hyteria, etc.).

L'Ecosystème considéré consistant en : tout système cellulaire constitué de cellules sensibles aux agents infectieux susceptibles de se trouver dans une sphère pathologique où est effectué le prélèvement (le système pouvant être constitué d'un ou plusieurs types de cellules selon les besoins) + mycoplasmes comme agent potentialisateur de la multiplication virale en culture cellulaire.

Les travaux et expérimentations ayant abouti à la présente invention ont essentiellement porté sur deux sphères pathologiques :
- uro-génitale avec le HSV (cellules Véro) et Chlamydiae Trachomatis (cellules Mac Coy) ;
- pulmonaire avec le RSV (cellules Hep-2) et Chlamydiae Twar (cellules Hela) ; et sur divers mycoplasmes :
- ceux ayant un tropisme particulier pour la sphère uro-génitale (Uu : Uréaplasma uréalyticum, Mh : Mycoplasma hominis) et pulmonaire (MP : Mycoplasma pneumoniae)
- ceux qui sont les plus répandus (MF : M. Fermentans, MS : M. Salivarium, MG : M. Genitalium, MO : M. Orale, MHy : M. Hyorinis, MA : M. Arginini, MPi : M. Pirum, MN : M. Nou (souche MPi), etc.

Pour cette raison, dans la description de l'exemple de mise en oeuvre du procédé de l'invention qui est faite ci-après, l'application des cellules mycoplasmisées porte essentiellement sur la prévention et la détection des MST (Maladies Sexuellement Transmissibles), des cancers du col, et de toutes maladies dont l'agent infectieux est potentialisé par un mycoplasme.

### I - Mycoplasmisation des cellules

Les cellules sont cultivées dans des boîtes ; elles sont mycoplasmisées à confluence. Les mycoplasmes utilisés pour infecter ces cellules sont préalablement amenés en phase de croissance dans leur milieu (milieu de culture pour mycoplasmes additionné d'un indicateur coloré).

Les cultures de cellules sont infectées dans un milieu compatible avec la double viabilité : cellule et mycoplasme.

La croissance est déterminée par une modification de la couleur du milieu.

Les boîtes sont vidées de leur surnageant, puis infectées sous un inoculum du milieu contenant les mycoplasmes, additionné par du milieu de culture cellulaire. Les boîtes sont incubées environ une heure sous agitation lente, entre 36 degrés C et 39 degrés C, puis le milieu est complété par du milieu de culture cellulaire. Les boîtes complémentées au milieu de culture sont contrôlées au microscope, puis sont trypsinées 2 à 3 jours après l'infection, et cultivées dans leur milieu habituel.

Les signes de contamination sont fréquemment visibles en microscopie optique (pourtour cellulaire et morphologie générale), mais les cellules sont généralement viables et peuvent être cultivées.

### II - Détection des mycoplasmes

Différents systèmes peuvent être utilisés :
- Sonde nucléique chaude ou froide spécifique des mycoplasmes permettant la détection du DNA des mycoplasmes par hybridation moléculaire ;
- Anticorps monoclonaux permettant la détection d'un antigène spécifique de mycoplasme par révélation immunoenzymatique sur membrane en utilisant un monoclonal dirigé contre cet antigène et un anti-souris conjugué à la péroxydase ;
- Culture en milieu solide ou liquide permettant la détection directe ou indirecte des mycoplasmes.

### III - Mise en évidence de la potentialité du mycoplasme comme cofacteur augmentant la sensibilité de test de diagnostic "in vitro".

L'augmentation de signal dû à la présence de mycoplasmes dans des cultures cellulaires est mise en évidence par comparaison entre les niveaux d'infection obtenus avec cellules mycoplasmisées et cellules homologues normales (non infectées par des mycoplasmes). Les infections ont lieu simultanément avec un inoculum homogène, sur des cellules à même état de confluence.

Les exemples donnés ci-dessous portent sur les virus HSV et RSV, ainsi que sur le Chlamydiae.

### - 1/ Infection

Les infections sont réalisées selon un protocole standard. Les cellules (mycoplamisées et non mycoplasmisées témoin) sont vidées de leur surnageant et inoculées sous lente agitation pendant 1 heure à 37 degrés C par l'agent infectieux sous faible volume (dilution dans du milieu de culture cellulaire sans sérum, appelé supplément). Du supplément est ensuite ajouté, puis les cellules sont incubées à 37 degrés C pendant 3 Jours.

### - 2/ Révélation de l'infection

Elle est faite par marquage immunologique sur membrane d'un antigène spécifique de l'agent infectieux, détecté par un anticorps monoclonal dirigé contre cet antigène, et marqué à une enzyme, elle-même révélée par un substrat chromogène. L'intensité de la coloration, exprimée en + sur une échelle allant de 0 à 4+ (+/- représentant la valeur maximum assimilable à du bruit de fond) est proportionnelle à la quantité d'antigènes présents, et permet d'évaluer l'intensité de l'infection. La sensibilité de cette technique immunologique a été confirmée par la classique détermination du titre infectieux par titrage en dilution limité sur microplaque.

Abréviations utilisées dans les exemples donnés dans les tableaux suivants : DI = détection immunologique, avec contrôle négatif sans agent infectieux, afin de voir si la cellule mycoplasmisée ne cross-réagit pas avec le monoclonal. DM = détection de mycoplasme.

**Tableau I -**

| HSV | | | |
|---|---|---|---|
| Cellule | DI/HSV+ | DI/HSV- | DM |
| Véro Témoin | + | +/- | - |
| Véro-MP | 2+ | +/- | + |
| Véro-MH | 4+ | +/- | + |
| Véro-MF | 2+ | +/- | + |
| Véro-UU | 4+ | +/- | + |
| Véro-MS | 3+ | +/- | + |

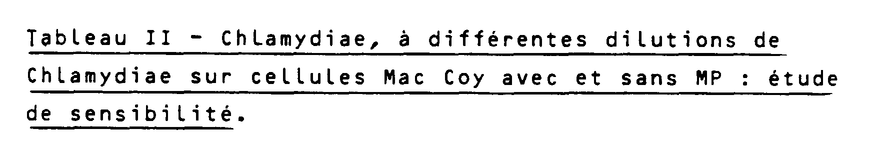

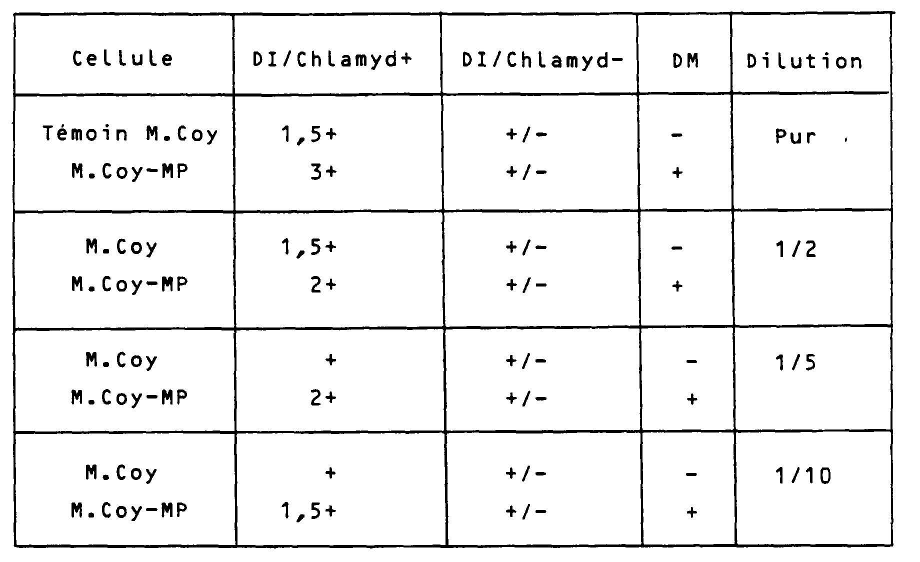

Ce dernier tableau est particulièrement significatif dans le cas du Chlamydiae. Il montre qu'un résultat douteux, proche de +/-, peut devenir beaucoup plus net et franchement positif.

**Tableau III-**

| RSV | | | |
|---|---|---|---|
| Cellule | DI/RSV+ | DI/RSV- | DM |
| Hep-2 | + | +/- | - |
| Hep-2-MP | 2,5+ | +/- | + |

Après l'exposé qui précède, on comprend que selon la présente invention, l'activité des mycoplasmes comme cofacteur d'infection virale ou bactérienne peut être exploitée en biotechnologie pour amplifier la réponse cellulaire, dans des kits de diagnostic utilisant des cultures cellulaires mycoplasmisées.

D'autre part, le choix du mycoplasme pour la sphère pathologique concernée, est particulièrement important. Il donne toute sa valeur clinique au terme "cofacteur". L'exemple exposé à propos du HSV montre, en effet, que pour potentialiser au maximum la culture, on aura tout intérêt utiliser des cellules Véro mycoplasmisées avec Uu ou Mh, qui sont justement et également des germes de la sphère uro-génitale, fréquemment associés voire incriminés dans de nombreuses pathologies de cette région.

## Revendications

1. Procédé d'amplification des agents infectieux en diagnostic "in vitro" sur cultures cellulaires, caractérisé par l'utilisation d'écosystème représentatif de la sphère pathologique concernée, reconstitué in vitro avec des cellules permissives pour lesdits agents, additionnées de mycoplasmes vivants comme élément de potentialisateur de la multiplication virale ou bactérienne en culture cellulaire de virus (par exemple: HSV, RSV) et de bactéries (par exemple : Chlamydiae, Legionella, Hystéria).

2. Procédé d'amplification des agents infectieux en diagnostic "in vitro" sur cultures cellulaires, selon la revendication 1, caractérisé en ce que l'on infecte lesdites cultures cellulaires "in vitro" avec des mycoplasmes vivants permettant d'induire une réponse cellulaire potentialisée par la présence du ou des mycoplasmes vivants utilisés.

3. Procédé selon la revendication 2, caractérisé en ce que l'on utilise pour la recherche et l'amplification des agents infectieux, des cellules et des mycoplasmes vivants choisis en fonction de la sphère où le prélèvement est effectué, et des agents infectieux recherchés.

4. Procédé suivant l'une des revendications 2 ou 3, caractérisé en ce que les cultures de cellules sont infectées, dans un milieu compatible avec la double viabilité cellule et mycoplasme vivant, avec des mycoplasmes vivants préalablement amenés en phase de croissance dans leur milieu de culture.

5. Procédé selon la revendication 4, suivant lequel les cellules sont cultivées dans des boîtes de culture, caractérisé en ce que leur infection ou mycoplasmisation est opérée de la manière suivante : les boîtes sont vidées de leur surnageant, puis infectées en milieu constitué de milieu contenant les mycoplasmes vivants et du milieu de culture cellulaire et ensuite incubées environ une heure sous agitation lente, entre 36 degrés C et 39 degrés C ; les boîtes complémentées au milieu de culture sont contrôlées au microscope ; elles sont trypsinées 2 à 3 jours après l'infection, et cultivées dans leur milieu habituel.

6. Procédé selon l'une quelconque des revendications 2 à 5, caractérisé en ce que l'augmentation de signal dû à la présence de mycoplasmes vivants dans des cultures cellulaires, est mise en évidence par comparaison entre les niveaux d'infection obtenus avec cellules mycoplasmisées et cellules homologues normales ; les infections ayant lieu simultanément avec un inoculum homogène, sur des cellules à même état de confluence.

7. Procédé suivant la revendication 6, caractérisé en ce que la révélation de l'infection est obtenue par marquage immunologique sur membrane d'un antigène spécifique de l'agent infectieux, détecté par un anticorps monoclonal dirigé contre cet antigène, et marqué à une enzyme, elle-même révélée par un substrat chromogène ; l'intensité de la coloration, exprimée en + sur une échelle allant de 0 à 4+ (+/- représentant la valeur maximum assimilable à du bruit de fond), est proportionnelle à la quantité d'antigènes présents et permet d'évaluer l'intensité de l'infection ; le niveau de détection étant confirmé par titrage classique du pouvoir infectieux par dilution limité sur microplaque.

8. Procédé selon l'une des revendications 6 ou 7, appliqué à la révélation d'une infection virale ou bactérienne, caractérisé en ce que l'infection de la culture cellulaire, préalable à ladite révélation, est réalisée selon un protocole standard ; les cellules (mycoplasmisées et non mycoplasmisées témoin) sont vidées de leur surnageant, et inoculées sous lente agitation pendant une heure à 37 degrés C par l'agent infectieux sous faible volume (dilution dans du milieu de culture cellulaire sans sérum, appelé supplément), du supplément est ensuite ajouté, puis les cellules sont incubées à 37 degrés C pendant 3 jours.

## Patentansprüche

1. Verfahren zur Verstärkung von infektiösen Wirkstoffen bei der "in vitro"-Diagnose auf Zellkulturen, gekennzeichnet durch die Verwendung eines für den betreffenden pathologischen Bereich repräsentativen Ökosystems, das in vitro mit gegenüber den Wirkstoffen toleranten Zellen nachgebildet wird, wobei lebende Mycoplasmen als Potenzierungselement für die Vervielfachung der Viren oder Bakterien in der Zellkultur für Viren (zum Beispiel: HSV, RSV) und für Bakterien (zum Beispiel: Chlamydiae, Legionella, Listerria) hinzugefügt werden.

2. Verfahren zur Verstärkung von infektiösen Wirkstoffen bei der "in vitro"-Diagnose auf Zellkulturen nach Anspruch 1, dadurch gekennzeichnet, daß die Zellkulturen "in vitro" mit lebenden Mycoplasmen infiziert werden, die eine Induzierung einer potenzierten Zellreaktion durch die Anwesenheit des oder der verwendeten lebenden Mycoplasmen ermöglichen.

3. Verfahren nach Anspruch 2, dadurch gekennzeichnet, daß für die Untersuchung und die Verstärkung der infektiösen Wirkstoffe Zellen und lebende Mycoplasmen verwendet werden, die in Abhängigkeit von dem Bereich, in dem der Abstrich vorgenommen wird, sowie in Abhängigkeit von den untersuchten infektiösen Wirkstoffen ausgewählt werden.

4. Verfahren nach einem der Ansprüche 2 oder 3, dadurch gekennzeichnet, daß die Zellkulturen in einem Medium, das mit der zweifachen Lebensfähigkeit von Zelle sowie lebendem Mycoplasma vereinbar ist, mit lebenden Mycoplasmen infiziert werden, die vorher in ihrem eigenen Kulturmedium in eine Wachstumsphase versetzt wurden.

5. Verfahren nach Anspruch 4, bei welchem die Zellen in Kulturschalen gezüchtet werden, dadurch gekennzeichnet, daß ihre Infektion oder Mycoplasmierung wie folgt vorgenommen wird: aus den Schalen wird der Überstand entfernt, dann werden sie in dem Medium, welches aus dem die lebenden Mycoplasmen enthaltenden Medium und aus Zellkulturmedium gebildet wird, infiziert und anschließend bei einer Temperatur zwischen 36 °C und 39 °C etwa eine Stunde lang unter langsamer Bewegung inkubiert; die komplementären Schalen mit dem Kulturmedium werden unter dem Mikroskop kontrolliert; 2 bis 3 Tage nach der Infektion werden sie mit Trypsin behandelt und in ihrem üblichen Medium kultiviert.

6. Verfahren nach einem der Ansprüche 2 bis 5, dadurch gekennzeichnet, daß die auf die Anwesenheit von lebenden Mycoplasmen in den Zellkulturen zurückzuführende Signalerhöhung durch den Vergleich zwischen den Infektionsniveaus hervorgehoben wird, die mit mycoplasmierten Zellen und mit homologen normalen Zellen erreicht werden; wobei die Infektionen mit einem homogenen Inokulum auf Zellen mit dem gleichen Konfluenz-Zustand gleichzeitig stattfinden.

7. Verfahren nach Anspruch 6, dadurch gekennzeichnet, daß die Entdeckung der Infektion durch eine immunologische Membran-Markierung eines spezifischen Antigens des infektiösen Wirkstoffs erreicht wird, das von einem gegen dieses Antigen gerichteten monoklonalen Antikörper entdeckt und mit einem Enzym markiert wird, das wiederum durch ein chromogenes Substrat erkennbar wird; die Intensität der Färbung, die als + auf einer Skala von 0 bis 4+ ausgedrückt wird, (wobei +/- den maximalen, mit einem Grundwert vergleichbaren Wert darstellt), ist proportional zur Menge der vorhandenen Antigene und ermöglicht die Bewertung der Intensität der Infektion; das Erfassungsniveau wird mittels klassischer Titration der Infektionsstärke über begrenzte Verdünnung auf Mikroplatten bestätigt.

8. Verfahren nach einem der Ansprüche 6 oder 7, angewendet auf die Entdeckung einer viralen oder bakteriellen Infektion, dadurch gekennzeichnet, daß die Infektion der Zellkultur vor der Entdeckung nach einem Standardprotokoll durchgeführt wird; von den Zellen (mycoplasmierte Zellen und nichtmycoplasmierte Kontrollzellen) wird der Überstand entfernt und sie werden unter langsamen Bewegungen eine Stunde lang bei 37 °C mit einer geringen Menge des infektiösen Wirkstoffs inokuliert (Verdünnung im Zellkulturmedium ohne Serum, genannt Supplement), anschließend wird das Supplement hinzugefügt und dann die Zellen drei Tage lang bei 37 °C inkubiert.

## Claims

1. Method of amplifying infectious agents for *in vitro* diagnosis in cell cultures, characterised by the use of an ecosystem representing the pathological sphere concerned, reconstituted *in vitro* with cells receptive to the said agents, with the addition of living mycoplasmas as an element for facilitating viral or bacterial multiplication in a cell culture of viruses (e.g. HSV, RSV) and bacteria (e.g. Chlamydiae, Legionella, Listerria).

2. Method of amplifying infectious agents for *in vitro* diagnosis in cell cultures according to claim 1, characterised in that the cell cultures are infected *in vitro with* living mycoplasmas so as to induce a cell response facilitated by the presence of the living mycoplasma(s) used.

3. Method according to claim 2, characterised in that for the research and amplification of infectious agents, cells and living mycoplasmas which are selected according to the sphere where sample is carried out and infectious agents being sought are used.

4. Method according to either of claims 2 or 3, characterised in that the cell cultures are infected in a medium compatible with the dual viability of the cell and living mycoplasma with living mycoplasmas previously brought into a growth phase in their culture medium.

5. Method according to claim 4, according to which the cells are cultivated in Petri dishes, characterised in that their infection or mycoplasmisation is carried out in the following manner: the Petri dishes are emptied of their floating surplus, are then infected with the medium containing the living mycoplasmas and with the cell culture medium, and are then incubated for about one hour with slow stirring, between 36 and 39°C; the dishes complemented with the culture medium are monitored under a microscope; trypsin is added 2 to 3 days after infection, and the dishes are cultivated in their usual surroundings.

6. Method according to any one of claims 2 to 5, characterised in that the signal increase due to the presence of living mycoplasmas in cell cultures is demonstrated by comparison between the levels of infection obtained with cells to which mycoplasma has been added and normal homologous cells; the infections taking place simultaneously with a homogeneous inoculant on cells at the same stage of confluence.

7. Method according to claim 6, characterised in that disclosure of the infection is obtained by immunological marking on a membrane of a specific antigen of the infectious agent, detected by a monoclonal antibody directed against this antigen, and marked by an enzyme, itself disclosed by a chromogenic substrate; the intensity of colouration, expressed in + on a scale from 0 to 4+ (+/- representing the maximum value comparable to background noise), is proportional to the quantity of antigens present and makes it possible to evaluate the intensity of the infection; the level of detection being confirmed by conventional titration of the infectious strength by limited dilution on a microchip.

8. Method according to either of claims 6 or 7 applied to the disclosure of a viral or bacterial infection, characterised in that the infection of the cell culture, prior to disclosure, is effected according to a standard protocol; the cells (both those with mycoplasma and those without and acting as a control) are emptied of their floating surplus and are inoculated with slow stirring for one hour at 37°C by the infectious agent in a small volume (dilution in cell culture medium without serum, referred to as supplement), supplement is then added, then the cells are incubated at 37°C for 3 days.
